# EUROPEAN PATENT SPECIFICATION

(11) **EP 3 624 868 B1**
(45) Date of publication and mention of the grant of the patent: **09.11.2022**
(21) Application number: 18731588.2
(22) Date of filing: 18.05.2018
(51) Int. Cl.: A61M 60/824

(54) **CENTER ROD MAGNET**
KOLBENSTANGENMAGNET
AIMANT DE TIGE CENTRALE

(30) Priority: 19.05.2017 US 201762508543 P
(43) Date of publication of application: 25.03.2020
(73) Proprietor: Heartware, Inc., Miami Lakes, FL 33014 (US)
(72) Inventor: SHAMBAUGH, Charles R., Coral Gables, Florida 33134 (US); TASKIN, Mustafa Ertan, Cooper City, Florida 33024 (US); MACK, Stanley P., Pinellas Park, Florida 33782 (US); LAROSE, Jeffrey A., Raleigh, North Carolina 27614 (US)
(74) Representative: Zimmermann & Partner Patentanwälte mbB
(86) International application number: PCT/US2018/033328
(87) International publication number: WO 2018/213666

(56) References cited:
- WO-A1-2006/066388
- WO-A1-2008/152425
- WO-A1-2009/099653
- WO-A1-2011/160056
- CN-U- 203 842 087
- US-A- 5 211 546

## Description

### TECHNICAL FIELD

The present invention relates to rotors for use in blood pumps and to blood pumps having such rotors. US 5,211,546 A describes an axial flow blood pump with hydrodynamically suspended rotor. WO 2006/066388 A1 describes an improved pump and method. WO 2011/160056 A1 describes a hydrodynamic chamfer thrust bearing.

### BACKGROUND

In certain disease states, the heart lacks sufficient pumping capacity to maintain adequate blood flow to the body's organs and tissues. For example, conditions such as ischemic heart disease and hypertension may leave the heart unable to fill and pump efficiently. This condition, also called congestive heart failure, may lead to serious health complications, including respiratory distress, cardiac asthma, and even death. In fact, congestive heart failure is one of the major causes of death in the Western World.

The inadequacy of the heart can be alleviated by providing a mechanical blood pump, also referred to as a ventricular assist device ("VAD"), to supplement the pumping action of the heart. VADs may be used to assist the right ventricle, left ventricle, or both. For example, a VAD may assist the left ventricle by mechanically pumping oxygenated blood from the left ventricle into the aorta.

One form of VAD includes an axial flow pump. In an axial flow pump, blood is transported through a chamber from an inlet to an outlet a path substantially parallel to the axis of rotation of a rotor disposed in the chamber. The rotor has blades that perform work on the fluid causing it to flow toward the outlet. As shown, for example, in United States patents 7,959,551 an axial flow rotor may be supported within the chamber by bearings separate from the rotor itself and driven by stator coils mounted to the pump and arrayed around the rotor. The stator coils generate a rotating magnetic field that interacts with the rotor to rotate the rotor around its axis. Another axial flow blood pump, shown in United States patent 7,934,909 uses a system of multiple magnetic bearings and hydrodynamic bearings to support and position the rotor within the chamber. These systems require elements in the flow path additional to the rotor hub and blades. These additional elements can impede the flow of blood through the pump and can cause thrombus to form within the pump.

Another type of blood pump, described in United States Patent 7,699,586 ("the '586 Patent)uses a rotor with wide blades having hydrodynamic bearing surfaces on the tip surfaces of the blades. Upon rotation of the rotor, the hydrodynamic interaction between the bearing surfaces on the tips of the blade and the chamber wall suspends the rotor in the chamber and maintains the axis of the rotor coaxial with the chamber. Certain embodiments of the rotor shown in the '586 patent have permanent magnets embedded in the blades of the rotor. These permanent magnets interact with the rotating magnetic field generated by the stator to spin the rotor about its axis. Magnetic interaction between the magnets and a ferromagnetic element incorporated in the stator holds the rotor in a desired position along the axis. However, such an arrangement requires assembly of multiple parts, with precise location of each magnet and precisely equal magnetization of the individual magnets to prevent imbalanced forces on the rotor, which can be challenging. To avoid these challenges, some wide-blade axial flow rotors have been made as a unitary body formed of a ferromagnetic material, which has a permanent magnetization transverse to the rotor's axis. However, the ferromagnetic materials from which such rotors are made must not only be ferromagnetic but also biocompatible and wear resistant. Materials, such as platinum alloys, that can satisfy both of these requirements are expensive and difficult to manufacture. Moreover, a magnetic wide-blade rotor typically is made with an even number of blades, most commonly four blades, to assure balanced operation.

### SUMMARY

Herein is disclosed the pump rotor as defined in the appended claims.

The present invention advantageously provides for a pump rotor including a hub defining a major longitudinal axis. A magnet is disposed within the hub along the major longitudinal axis. A plurality of rotor blades project outwardly from the hub away from the longitudinal axis and are spaced apart from one another in a circumferential direction around the longitudinal axis. Each of the plurality of rotor blades define a hydrodynamic bearing at an outer extremity thereof remote from the hub. The plurality of rotor blades define a plurality of flow channels. Each of the plurality of rotor blades is configured to drive a fluid through the flow channels upon rotation of the rotor around the axis. The magnet is a unitary solid and is coaxial with the hub, the magnet being radially magnetized and defining a plurality of radial poles, the magnet being the only magnetic component within the rotor.

In another aspect of this embodiment, the plurality of rotor blades define a collective area at an outer periphery of the rotor remote from the hub, and wherein the flow channels define a collective area at the outer periphery, and wherein the collective area defined by the plurality of rotor blades at the outer periphery is greater than the collective area defined by the flow channels at the outer periphery.

In another aspect of this embodiment, the magnet is cylindrical.

In another aspect of this embodiment, the hub includes tapered end portions and an intermediate portion disposed between the end portions, the intermediate portion houses the magnet and the rotor blades extend from the intermediate portion.

In another aspect of this embodiment, the magnet includes neodymium.

In another aspect of this embodiment, the plurality of rotor blades and the hub are non-ferromagnetic.

In another aspect of this embodiment, the plurality of rotor blades and hub are made from a polymer material.

In another aspect of this embodiment, the plurality of rotor blades and hub are made from a biocompatible material and the magnet includes a non-biocompatible material.

In another embodiment, a blood pump includes a flow chamber defining an axis. A motor stator having stator coils is disposed about the flow chamber. A rotor includes a hub defining a major longitudinal axis. A magnet is disposed within the hub along the major longitudinal axis. A plurality of rotor blades project outwardly from the hub away from the longitudinal axis and are spaced apart from one another in a circumferential direction around the longitudinal axis. Each of the plurality of rotor blades define a hydrodynamic bearing at an outer extremity thereof remote from the hub. The plurality of rotor blades define a plurality of flow channels. Each of the plurality of rotor blades is configured to drive a fluid through the flow channels upon rotation of the rotor around the axis. The stator coils are configured to generate a magnetic field within the flow chamber rotating about the axis of the flow chamber. The rotating magnetic field interacts with the magnet of the rotor to drive the rotor about the axis thereof.

In another aspect of this embodiment, the motor stator includes a back-iron and wherein the magnet and back-iron are passively attracted to each other and cooperate to restrain the rotor from axial displacement within the flow chamber during operation.

In another aspect of this embodiment, the magnet is enclosed within the rotor.

In another aspect of this embodiment, the magnet is sealed within the rotor.

In another aspect of this embodiment, the plurality of rotor blades are non-ferromagnetic.

In another aspect of this embodiment, the plurality of rotor blades define a collective area at an outer periphery of the rotor remote from the hub, and wherein the flow channels define a collective area at the outer periphery, and wherein the collective area defined by the plurality of rotor blades at the outer periphery is greater than the collective area defined by the flow channels at the outer periphery.

In another aspect of this embodiment, the magnet is a unitary solid and is coaxial with the hub, the magnet being radially magnetized and defining a plurality of radial poles.

In another aspect of this embodiment, the plurality of rotor blades and hub are made from a polymer material.

In another aspect of this embodiment, the plurality of rotor blades and hub are made from a biocompatible material and the magnet includes a non-biocompatible material.

In yet another embodiment, a method of operating a blood pump includes generating a rotating magnetic field configured to rotate a rotor of the blood pump. The rotor includes a hub and a magnet disposed within the hub.

### BRIEF DESCRIPTION OF THE DRAWINGS

A more complete understanding of the present invention, and the attendant advantages and features thereof, will be more readily understood by reference to the following detailed description when considered in conjunction with the accompanying drawings wherein:
FIG. 1 is a perspective view of a rotor according to an embodiment of the present disclosure;
FIG. 2 is an elevational view of the rotor of FIG. 1;
FIG. 3A is a front view of the rotor of FIG. 1;
FIG. 3B is a sectional view taken along line B-B of FIG. 3A;
FIG. 3C is a sectional view taken along C-C of FIG. 2;
FIG. 4 is an exploded view of the rotor of FIG. 1;

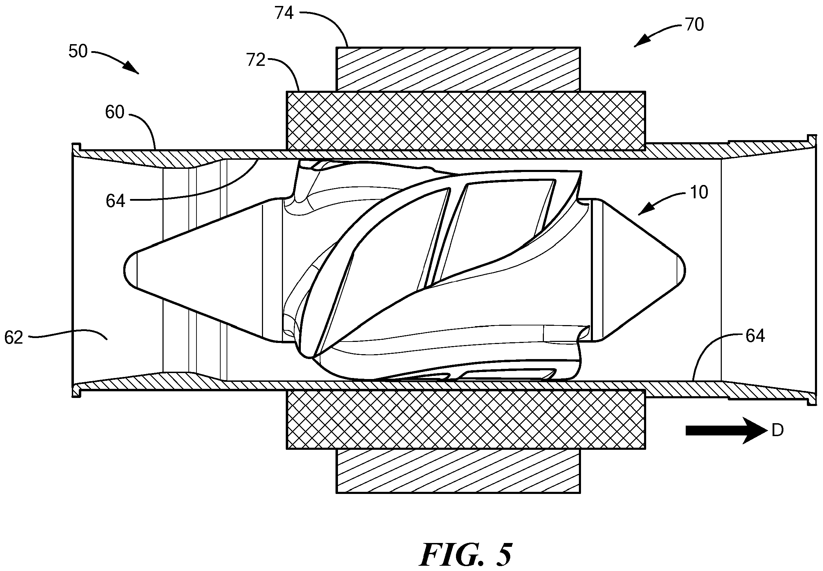

FIG. 5 is a schematic sectional view of a pump according to an embodiment of the disclosure including the rotor of FIG. 1;
FIG. 6 is a perspective view of a rotor according to another embodiment of the present disclosure;
FIG. 7 is an exploded view of the rotor of FIG. 6; and
FIG. 8 is a partially schematic view of a rotor according to another embodiment of the present disclosure.

### DETAILED DESCRIPTION

As used in this disclosure, the term "generally helical" refers to a feature which extends in the direction parallel to an axis and which curves in the circumferential direction around the axis over at least 50% of its extent in the direction along the axis. Also, as used herein, the terms "about" and "substantially" are intended to mean that slight deviations from absolute are included within the scope of the term so modified.

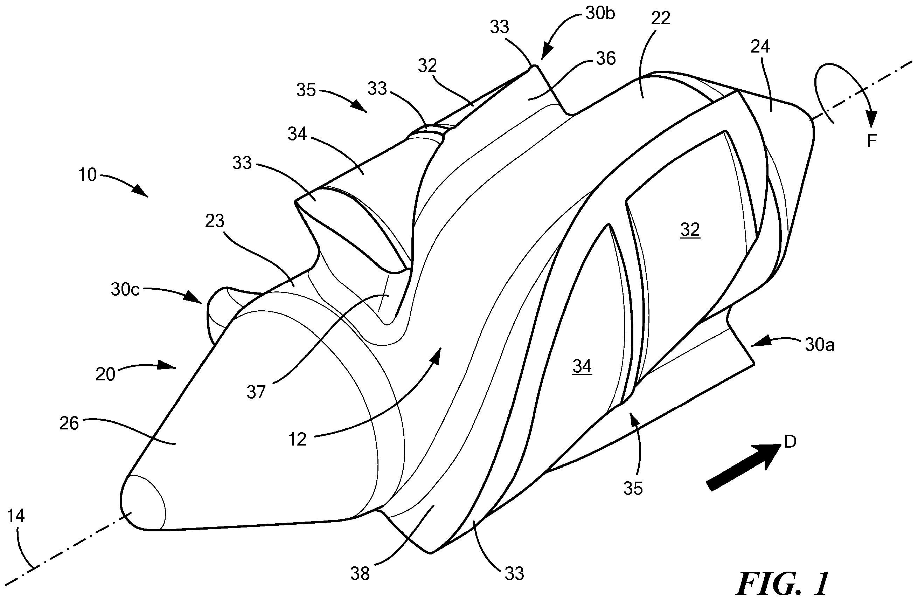

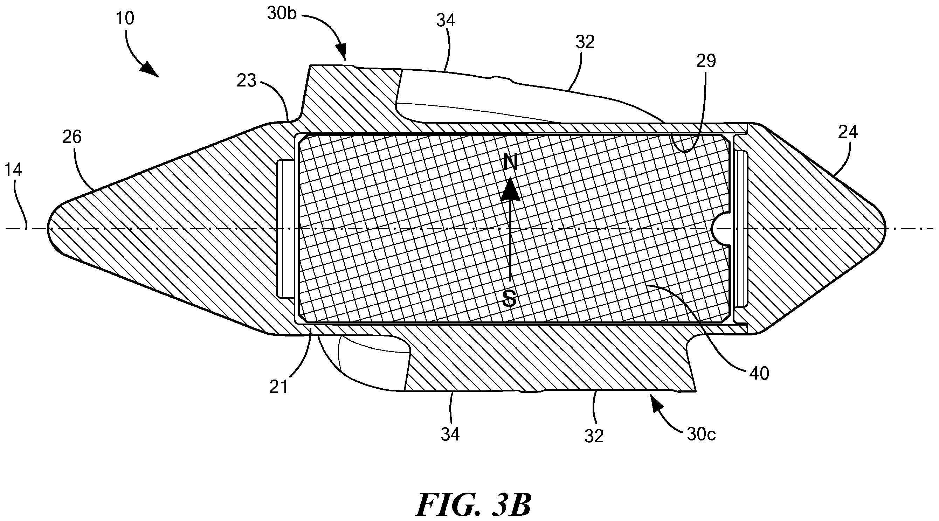

Referring now to the drawings in which like reference designators refer to like elements, there is a shown in FIGS. 1-4 a rotor or impeller 10 constructed in accordance with one embodiment of the present disclosure. Rotor 10 includes a hub 20, a plurality of rotor blades 30, and a magnet 40. Hub 20 defines a central body of rotor 10 and also defines a rotor axis or major longitudinal axis 14 about which rotor 10 rotates. Hub 20 includes end portions 24, 26 and an intermediate portion 22 disposed between end portions 24 and 26. The end portions 24, 26 are tapered solids of revolution, and intermediate portion 22 is substantially in the form of a body of revolution which may have a uniform cross-sectional dimension along its length, or which may vary in cross-sectional dimension. In this regard, intermediate portion 22 may be cylindrical, and end portions 24 and 26 may be conical as is depicted. Intermediate portion 22 is hollow and includes a sidewall 21 that defines an interior space surrounded by a wall surface 29 in the form of a surface of revolution about axis 14. As best shown in FIG. 3B, such interior space is sized to receive and house magnet 40.

The plurality of rotor blades 30 project from the hub 20. In the particular embodiment depicted, the plurality of blades 30 includes exactly three rotor blades 30a-c. Each blade 30 extends outwardly from hub 20 away from hub axis 14 to an outer extremity thereof remote from hub 20. More particularly, each blade 30 extends out of hub 20 in an outward radial or "spanwise" direction perpendicular to the axis 14. Each blade 30 also extends in a lengthwise or axial direction over a portion of the axial extent of hub 20 so that blades 30a-c are coextensive with one another in the axial direction. In the particular embodiment depicted, each blade 30 extends along the length of intermediate portion 22 and terminates adjacent end portions 24 and 26 of hub 20. In other words, blades 30a-c project outwardly from intermediate portion 22. However, in some embodiments, blades 30a-c may partially project outwardly from end portions 24 and 26 as well as from intermediate portion 22.

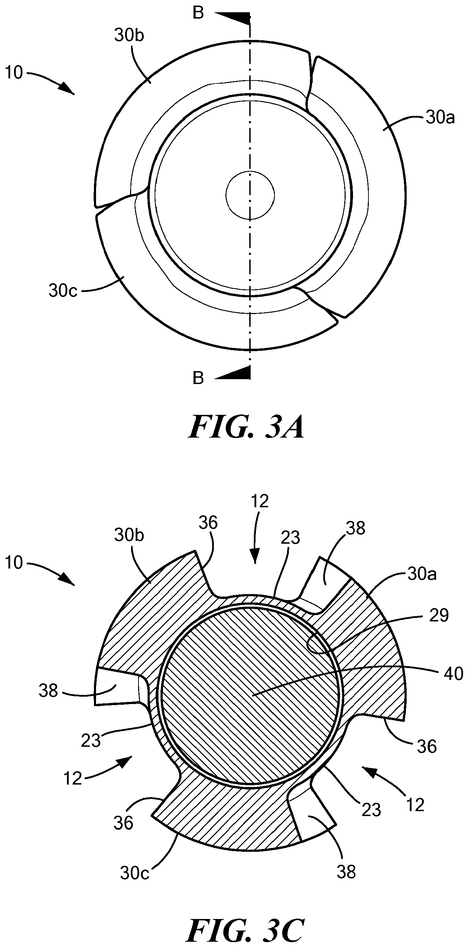

Each blade 30 defines generally helical surfaces 36, 38 that intersect the outer surface or floor surface 23 of intermediate portion 22 of hub 20. These helical surfaces 36, 38 are referred to as a pressure surface 36 and a suction surface 38, as shown in FIG. 3C. The pressure and suction surfaces 36, 38 are disposed at opposite sides of each blade 30 and converge with each other at inflow and outflow edges 37, 39 which are disposed at inflow and outflow ends of blades 30a-c. Pressure surface 36 faces forward, i.e., the circumferential direction in which the rotor spins as indicated by arrow F in FIG. 1, and suction surface 38 faces rearward, i.e., the circumferential direction opposite the forward direction. The arrow D in the drawings indicates the direction of flow from upstream to downstream.

Rotor blades 30a-c are evenly spaced apart from one another around axis 14 in forward and rearward circumferential directions. Thus, blades 30a-c define a plurality of flow channels 12 that extend between blades 30a-c and likewise in an axial direction along rotor axis 14. Such channels 12 are bounded by outer surface 23 of intermediate portion 22 and the pressure and suction surfaces 36, 38 of adjacent blades 30. In this regard, flow channels 12 are generally helical to correspond to the helical profile of pressure and suction surfaces 36 and 38.

Each blade 30 has a tip surface 35 intersecting with and extending between the pressure surface and suction surface 36, 38. Each tip surface 35 faces outwardly away from axis 14 and defines the outermost extremity or outer periphery of both blade 30 and the rotor 10 itself. These tip surfaces 35 define a collective surface area that is larger than a collective area defined by flow channels 12 at the outer periphery of rotor 10. In other words, each tip surface 35 of blades 30a-c is large as compared to empty space of flow channels 12 such that in the aggregate, the surface area defined by tip surfaces 35 is more extensive than the aggregate area of flow channels 12 taken at the periphery of rotor 10. In this regard, rotor 10 is characterized as a wide-blade or large-area rotor. Other exemplary wide-blade rotors are described in the heretofore referenced '586 Patent; U.S. Patent Nos. 7,972,122; 8,007,254; 8,419,609 and U.S. Publication No. 2015/0051438. The wide-blade configuration of rotor 10 allows rotor blades 30a-c to have hydrodynamic bearing surfaces at blade tips 35 that are capable of suspending rotor 10 within a pump housing during operation without the need for mechanical supports. Also, such a wide-blade configuration of rotor 10, particularly in combination with hydrodynamic bearings at blade tips 35, allows it to be extraordinarily compact. For example, the maximum diameter of rotor 10 at blades 30a-c may be about 0.5 inches (12.7 mm) and have an overall length of about 0.86 inches (21.8mm).

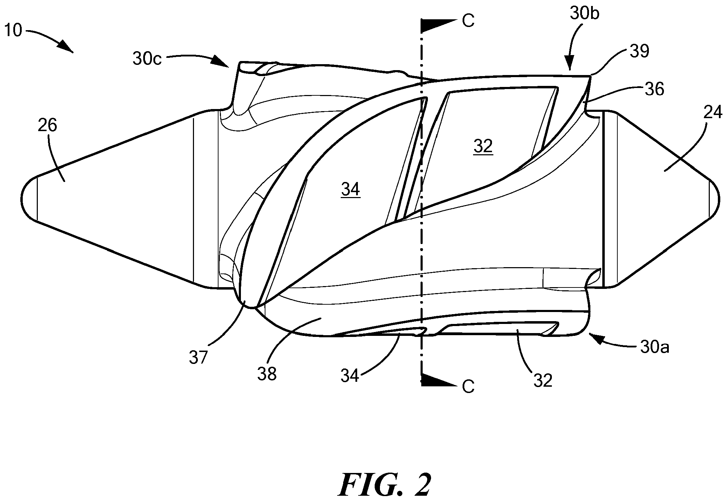

In the configuration shown in FIGS. 1 and 2, each tip surface 35 of rotor blades 30a-c includes a land surface 33, an upstream hydrodynamic bearing surface 34 and a downstream hydrodynamic bearing surface 32. Land surface 33 is in the form of a part of a surface of revolution around central axis 14. In the particular embodiment depicted, the surface of revolution is a circular cylinder so that the radius from axis 14 to a land surface 33 is uniform over the entire extent of rotor 10 and so that this radius is one-half the maximum diameter of rotor 10 at blades 30.

Each hydrodynamic bearing surface 32, 34 extends in the rearward circumferential direction from pressure surface 36 of its respective blade 30 and is bounded by and recessed radially relative to land surface 33. The recess of bearing surfaces 32, 34 is at a maximum at the forward edge of such surfaces where bearing surfaces 32, 34 meet pressure surface 36 of the blade 30. The recess of each bearing surface 32, 34 diminishes progressively in the rearward circumferential direction, so that each bearing surface 32, 34 merges smoothly into land surface 33 at the rearward edge of each bearing surface 32, 34.

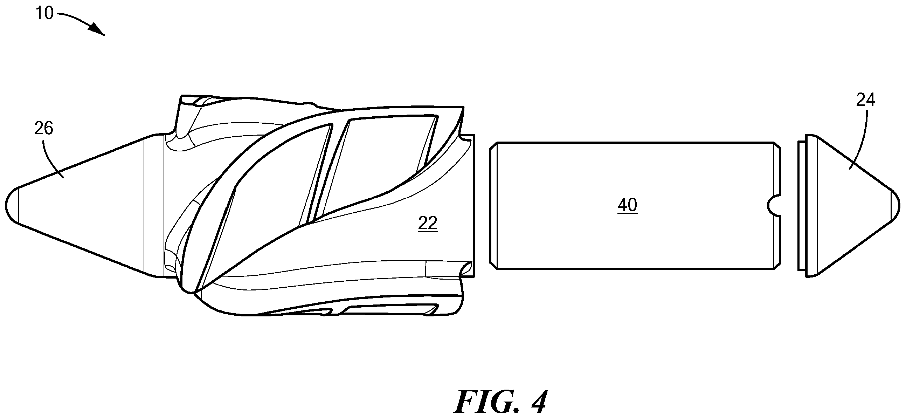

Referring now to FIGS. 3 and 4, in one configuration, magnet 40 is a permanent magnet and is a unitary solid of revolution. In this regard, magnet 40 may be a solid cylinder in which it is completely solid through its thickness, as shown. However, magnet 40 can also be an elongate tube with a hollow interior. Magnet 40 is sized to fit and be retained within the interior space of hub 20. In addition, magnet 40 is magnetized with a magnetic field direction transverse to its axis, and desirably perpendicular to its axis so as to have a plurality of radial poles. The magnet and has sufficient magnetic flux to rotate rotor 10 when a moving magnetic field is applied to such poles. In one configuration, magnet 40 is the only component having ferromagnetic properties within rotor 10, which simplifies the construction of rotor 10. Magnet 40 can be made from any magnetic material including non-biocompatible materials and biocompatible materials. For example, magnet 40 may be made from neodymium and alloys thereof, or aluminum-cobalt-nickel alloys.

As best shown in FIG. 3B, magnet 40 is disposed within intermediate portion 22 of hub 20 and fixed thereto so that the axis of the magnet 40, and hence its center of mass, is aligned with the axis 14 of the rotor 10. Magnet 40 also extends from one end of intermediate portion 22 to the other end and terminates just short of end portions 24 and 26. However, in some embodiments, magnet 40 may extend into the end portions 24, 26. In addition, magnet 40 is disposed within hub 20 so that magnet 40 is aligned with rotor blades 30a-c in the axial direction and is generally coaxial with axis 14.

Magnet 40 is a permanent magnet made from ferromagnetic materials that may or may not be biocompatible. However, the biocompatibility magnet 40 is of no import when implanted within a patient because such magnet 40 is embedded within rotor 10 which itself has a biocompatible exterior. In addition to having a biocompatible exterior, rotor 10 is non-ferromagnetic. As discussed above, magnet 40 may be the only magnetic component within rotor 10. In other words, hub 20 and rotor blades 30a-c are made from non-ferromagnetic materials and are either made from a biocompatible material or made from a non-biocompatible material with a biocompatible coating. For example, rotor 10 may be made from a biocompatible polymer material, such as silicone polymers, fluoroalkylsiloxane polymers or polyphosphazenes. Such polymer material can be molded over magnet 40. Alternatively, a polymeric rotor 10 can be molded separately from magnet 40 and machined so as to form the interior space within hub 20 for magnet 40. A separately molded end portion 24, such as that shown in FIG. 4, may then be welded, such as by friction welding, or otherwise fixed to intermediate portion 22 to seal magnet 40 therein.

Rotor 10 can also be made from non-ferromagnetic metals, such as nonmagnetic stainless steel or titanium, or non-ferromagnetic ceramics, such as pyrolytic carbon, aluminum oxide, and zirconium oxide, for example. Furthermore, rotor 10 may have a biocompatible coating, such as a parylene, silicone, chromium nitride, or titanium nitride coating, for example. Rotor 10 can be made from a combination of the materials described above, but overall the rotor itself, regardless of the materials selected, is non-ferromagnetic. In this regard, the selection of materials is numerous and can be selected to control costs and/or optimize performance without the additional concern of providing magnetization as such is provided by center magnet 40.

A pump 50 according to one embodiment of the present invention includes a pump housing 60, motor stator 70, and rotor 10 as discussed hereinabove with reference to FIGS. 1-4. Housing 60 defines an interior bore or flow channel 62. Rotor 10 is disposed within flow channel 62 and an interior surface 64 of housing 60 surrounds tip surfaces 35 of rotor 10. The motor stator 70 is disposed around the exterior of housing 60. Rotor blades 30a-c are disposed directly between motor stator 70 and magnet 40. Motor stator 70 includes a set of coils 72 that are arrayed around the exterior of housing 60. Coils 72 may be of conventional construction. By way of example, coils 72 may be provided as three sets of diametrically opposed coils disposed at equal spacing around the circumference of housing 60. Motor stator 70 also includes a ferromagnetic component, referred to as a back-iron, which is associated with stator coils 72.

In operation, with pump 50 implanted in the body of mammalian subject, and with housing 60 connected into the circulatory system, for example, in the conventional manner for a VAD, coils 72 are actuated to provide a magnetic field directed transverse to rotor axis 14 to cause such field to rotate rapidly around axis 14. Such magnetic field interacts with the radial poles of magnet 40 disposed within rotor 10 to rotate magnet 40 and, consequently, rotor 10 itself along with the magnetic field.

Rotor 10 also passively interacts with back-iron 74 of motor stator 70. In this regard, the permanent magnetism of back-iron 74 and center magnet 40 results in a magnetic attraction that resists axial displacement of rotor 10 within flow channel 62 that may be caused by pressure head gravity, or both. Also, while rotor 10 is rotated, a thin film of blood between hydrodynamic bearing surfaces 32, 34 and interior surface 64 of housing 60 is formed which maintains rotor 10 coaxial with housing 60 so that rotor 10 does not contact interior surface 64 due to radial movement, transverse to the axis 14 of rotor 10, or due to tilting of axis 14 relative to housing 60. Thus, hydrodynamic bearings 32, 34 of blades 30a-c in conjunction with the axial alignment provided by the attraction of center magnet 40 and back-iron 74 eliminate the need for mechanical suspension systems to stabilize rotor 70 during operation. This allows flow channel 62 to be free and clear of obstructions other than rotor 10 itself.

The rotor 10 as discussed above offers significant advantages. For example, because the blades 30 of the rotor 10 are formed from non-ferromagnetic materials, the blades 30 do not introduce imbalanced magnetic forces, and the rotor 10 can operate stably with three blades 30. A rotor 10 with three blades 30 and three channels having a given aggregate cross-sectional area, provides better flow conditions than a comparable four bladed rotor with four channels having the same aggregate cross-sectional area. Precise alignment between the axis of the magnet 40 and the axis 14 of the rotor is achieved in common manufacturing techniques. For example, the magnet can be formed to a body of revolution of precise dimensions by techniques such as machining or centerless grinding. The interior surface 29 of the wall surrounding the interior space can be formed to a surface of revolution having precise dimensions and precisely coaxial with the axis and with the land surfaces 33 of the blades, by machining or molding the interior surface.

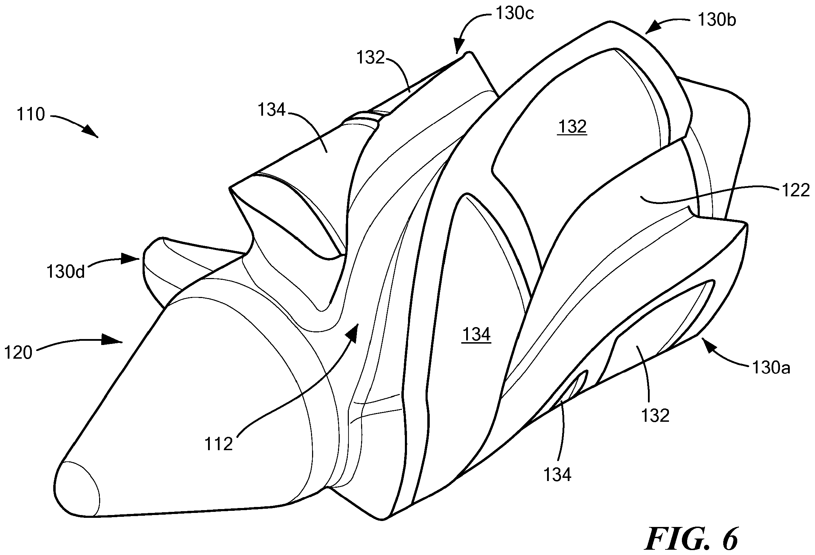

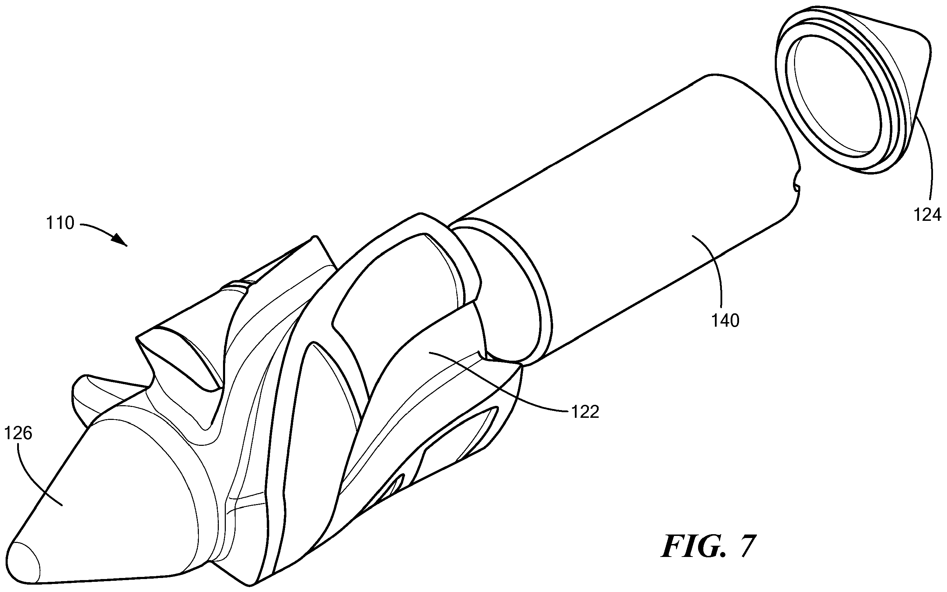

Other alternative embodiments of the aforementioned devices are contemplated. For example, FIGS. 6 and 7 depict an alternative embodiment rotor 110. Such rotor 110 is similar to rotor 10 in that it includes a hub 120, a center magnet 140 disposed within an intermediate portion 122 of hub 120, and a plurality of wide-blades 130 that define hydrodynamic bearing surfaces 132, 134 and flow channels 112 between such blades 130. Moreover, just as with rotor 10, the only magnetic component in rotor 110 is center magnet 140. However, rotor 110 differs in that it includes four rotor blades 130a-d, rather than three. In this regard, a center magnet, such as magnets 40 and 140, in conjunction with non-ferromagnetic blades, such as blades 30 or 130, allows any number of rotor blades to be utilized without concern that the number of blades selected would affect magnetic balancing.

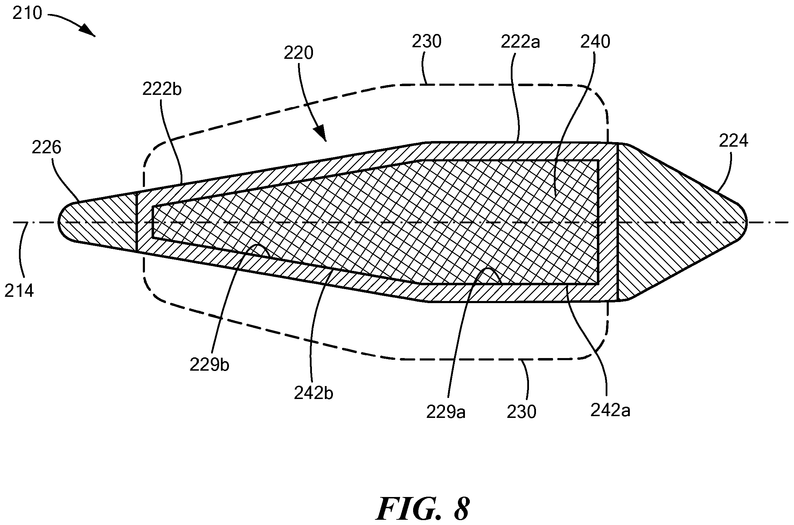

Another rotor 210 is shown in FIG. 8 according to a further embodiment of the present disclosure. Rotor 210 is similar to rotor 10 in that it includes a hub 220, rotor blades 230 (shown in phantom lines) extending from hub 220, and a center magnet 240. Such rotor blades 230 may be non-ferromagnetic and also have hydrodynamic bearing surfaces at their tips, as described above. As shown, hub 220 has a hollow intermediate portion 222 disposed between end portions 224 and 226. Intermediate portion 222 includes a first section 222a and a second section 222b that each partially define an interior space of hub 220. In this regard, first section 222a includes a first wall surface 229a in the form of a surface of revolution about rotor axis 214, and second section 222b includes a second wall surface 229b also in the form of a surface of revolution about rotor axis 214. Such surfaces 229a-b surround and define the interior space. In the particular embodiment depicted, first and second wall surfaces 229a-b define the interior space such that interior space has a uniform cross-sectional dimension along first wall surface 229a and a tapering cross-sectional dimension along second wall surface 229b. However, in other embodiments, first and second wall surfaces 229a-b may define the interior space such that the interior space has a tapering cross-sectional dimension along both the first and second wall surfaces 229a-b but where the cross-sectional dimension of the interior space tapers at a greater rate along second wall surface 229b than first wall surface 229a. It is also contemplated that intermediate portion 222 of hub 220 may have a single wall surface of revolution that defines an interior space that tapers along its entire length so as to form a frustoconical space, for example.

In the rotor embodiment depicted in FIG. 8, magnet 240 may have smaller but proportional dimensions relative to that of intermediate portion 222 such that magnet 240 can be disposed in the interior space defined by surfaces 229a-b and so that an axis defined by magnet 240 is coaxial with axis 214. Thus, magnet 240, may be a unitary solid of revolution in which a first section 242a thereof has a first cross-sectional dimension proportional to first section 222a of hub 220 and a second section 242b thereof has a second cross-sectional dimension proportional to second section 222b of hub 220.

In the embodiments discussed above, the rotor is constrained against axial movement relative to the pump chamber by magnetic attraction between the magnet and the back-iron incorporated in the stator. In other embodiments, the hydrodynamic bearing surfaces of the rotor may include hydrodynamic bearing surfaces arranged to provide axial thrust and thus constrain the rotor against axial movement relative to the pump chamber and stator. For example, as disclosed in U.S. Published Patent Application No. 2011/0311383, hydrodynamic bearing surfaces facing in a direction oblique to the axis may be provided on the blades so as to provide axial thrust in one direction. To provide full axial constraint, the oblique surfaces may include oblique surfaces facing in opposite axial directions.

Certain embodiments of the invention include:
Embodiment 1. A pump rotor, comprising:
   a hub having a major longitudinal axis;
   a magnet disposed within the hub along the major longitudinal axis; and
   a plurality of rotor blades projecting outwardly from the hub away from the axis and being spaced apart from one another in a circumferential direction around the axis, each of the plurality of rotor blades defining a hydrodynamic bearing at an outer extremity thereof remote from the hub, the plurality of rotor blades defining a plurality of flow channels and each of the plurality of rotor blades being configured to drive a fluid through the flow channels upon rotation of the rotor around the axis.
Embodiment 2. The rotor of Embodiment 1, wherein the plurality of rotor blades are non-ferromagnetic.
Embodiment 3. The rotor of Embodiment 1, wherein the plurality of rotor blades define a collective area at an outer periphery of the rotor remote from the hub which is greater than a collective area defined by the flow channels at the outer periphery.
Embodiment 4. The rotor of Embodiment 1, wherein the magnet is a unitary solid of revolution coaxial with the hub, the magnet being radially magnetized so as to have a plurality of radial poles.
Embodiment 5. The rotor of Embodiment 4, wherein the magnet is cylindrical.
Embodiment 6. The rotor of Embodiment 1, wherein the hub includes tapered end portions and an intermediate portion disposed between the end portions, the intermediate portion houses the magnet and the rotor blades extend from the intermediate portion.
Embodiment 7. The rotor of Embodiment 1, wherein the magnet includes neodymium.
Embodiment 8. The rotor of Embodiment 1, wherein the magnet is the only magnetic component disposed within the rotor.
Embodiment 9. The rotor of Embodiment 1, wherein the plurality of rotor blades and hub are made from a polymer material.
Embodiment 10. The rotor of Embodiment 1, wherein the plurality of rotor blades and hub are made from a biocompatible material and the magnet includes a non-biocompatible material.
Embodiment 11. A pump rotor, comprising:
   a hub having a major longitudinal axis;
   a magnet disposed within the hub along the major longitudinal axis and at the center mass of the hub; and
   a plurality of discrete rotor blades projecting outwardly from the hub away from the axis and each defining a hydrodynamic bearing at an outer extremity thereof remote from the hub, the plurality of discrete rotor blades being spaced apart from one another in a circumferential direction around the axis and defining a plurality of flow channels, the plurality of discrete rotor blades being configured to drive a fluid through the flow channels upon rotation of the rotor around the axis.
Embodiment 12. The rotor of Embodiment 11, wherein the plurality of discrete rotor blades are non-ferromagnetic.
Embodiment 13. The rotor of Embodiment 11, wherein in the plurality of discrete rotor blades include exactly three rotor blades.
Embodiment 14. The rotor of Embodiment 11, wherein the plurality of discrete rotor blades define a collective area at an outer periphery of the rotor greater than a collective area defined by the flow channels at the outer periphery.
Embodiment 15. The rotor of Embodiment 11, wherein the magnet is a unitary solid of revolution coaxial with the hub, the magnet being radially magnetized so as to have a plurality of radial poles.
Embodiment 16. The rotor of Embodiment 15, wherein the magnet is cylindrical.
Embodiment 17. The rotor of Embodiment 11, wherein the magnet is disposed within the hub so that the magnet is aligned with the plurality of discrete rotor blades in the axial direction.
Embodiment 18. The rotor of Embodiment 11, wherein the magnet includes neodymium.
Embodiment 19. The rotor of Embodiment 11, wherein the hub includes tapered end portions and an intermediate portion disposed between the end portions, the intermediate portion houses the magnet and the plurality of discrete rotor blades extend from the intermediate portion.
Embodiment 20. The rotor of Embodiment 11, wherein the plurality of discrete rotor blades and hub are made from a polymer material.
Embodiment 21. The rotor of Embodiment 11, wherein the rotor and hub are made from a biocompatible material and the magnet is made from a non-biocompatible material.
Embodiment 22. A blood pump comprising:
   a flow chamber having an axis;
   a motor stator having stator coils disposed about the flow chamber; and
   a rotor as in any one of Embodiments 1-22 disposed within the flow chamber with the axis of the rotor coaxial with the axis of the flow chamber;
   wherein the stator coils are operable to provide a magnetic field within the flow chamber rotating about the axis of the flow chamber, the rotating magnetic field interacting with the magnet of the rotor to drive the rotor about the axis thereof.
Embodiment 23. The pump of Embodiment 22, wherein the motor stator includes a back-iron and wherein the magnet and back-iron are passively attracted to each other so as to restrain the rotor from axial displacement within the flow chamber during operation.
Embodiment 24. A method of pumping blood comprising implanting a blood pump as embodied in Embodiment 23 within a body of a patient, connecting the pump to the circulatory system of the patient, and actuating the pump to assist blood flow within the circulatory system.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised.

## Claims

1. A pump rotor (10, 110, 210), comprising:
a hub (20, 120, 220) defining a major longitudinal axis;
a magnet (40, 140) disposed within the hub along the major longitudinal axis; and
a plurality of rotor blades (30, 130) projecting outwardly from the hub away from the longitudinal axis and being spaced apart from one another in a circumferential direction around the longitudinal axis, each of the plurality of rotor blades (30, 130) defining a hydrodynamic bearing (32, 34, 132, ,134) at an outer extremity thereof remote from the hub (20, 120, 220), the plurality of rotor blades (30, 130) defining a plurality of flow channels (12), each of the plurality of rotor blades (30, 130) being configured to drive a fluid through the flow channels upon rotation of the rotor around the axis, the magnet (40, 140) is a unitary solid and is coaxial with the hub (20, 120, 220), the magnet being radially magnetized and defining a plurality of radial poles, the magnet being the only magnetic component within the rotor.

2. The rotor (10, 110, 210) of Claim 1, wherein the plurality of rotor blades (30, 130) define a collective area at an outer periphery of the rotor remote from the hub (20, 120, 220), and wherein the flow channels (12) define a collective area at the outer periphery, and wherein the collective area defined by the plurality of rotor blades (30, 130) at the outer periphery is greater than the collective area defined by the flow channels (12) at the outer periphery.

3. The rotor (10, 110, 210) of Claim 1, wherein the magnet (40, 140) is cylindrical.

4. The rotor (10, 110, 210) of any one of Claims 1-3, wherein the hub (20, 120, 220) includes tapered end portions (24, 26) and an intermediate portion (22) disposed between the end portions, the intermediate portion houses the magnet (40, 140) and the rotor blades (30, 130) extend from the intermediate portion.

5. The rotor (10, 110, 210) of any one of Claims 1-4, wherein the magnet (40, 140) includes neodymium.

6. The rotor (10, 110, 210) of any one of Claims 1-5, wherein the plurality of rotor blades (30, 130) and the hub (20, 120, 220) are non-ferromagnetic.

7. The rotor (10, 110, 210) of any one of Claims 1-6, wherein the plurality of rotor blades (30, 130) and hub (20, 120, 220) are made from a polymer material.

8. The rotor (10, 110, 210) of any one of Claims 1-7, wherein the plurality of rotor blades (30, 130) and hub (20, 120, 220) are made from a biocompatible material and the magnet (40, 140) includes a non-biocompatible material.

9. The rotor (10, 110, 210) of any one of Claims 1-8, wherein the magnet (40, 140) is enclosed within the rotor.

10. The rotor (10, 110, 210) of Claim 9, wherein the magnet (40, 140) is sealed within the rotor.

11. The rotor (10, 110, 210) of any one of Claims 1-10, wherein the hub (20, 120, 220) is sized to be received within a human patient.

12. The rotor (10, 110, 210) of any one of Claims 1-11, wherein the rotor is sized to be received within an implantable blood pump (50).

## Patentansprüche

1. Pumpenrotor (10, 110, 210), umfassend:
eine Nabe (20, 120, 220), die eine Hauptlängsachse definiert;
einen Magneten (40, 140), der innerhalb der Nabe entlang der Hauptlängsachse angeordnet ist; und
eine Vielzahl von Rotorschaufeln (30, 130), die von der Nabe nach außen von der Längsachse weg vorstehen und in einer umlaufenden Richtung um die Längsachse herum voneinander beabstandet sind, wobei jede der Vielzahl von Rotorschaufeln (30, 130) ein hydrodynamisches Lager (32, 34, 132, 134) an einem äußeren Ende davon definiert, das von der Nabe (20, 120, 220) entfernt ist, wobei die Vielzahl von Rotorschaufeln (30, 130) eine Vielzahl von Strömungskanälen (12) definiert, wobei jede der Vielzahl von Rotorschaufeln (30, 130) konfiguriert ist, um bei Drehung des Rotors um die Achse herum ein Fluid durch die Strömungskanäle anzutreiben, wobei der Magnet (40, 140) ein einheitlicher Festkörper ist und mit der Nabe (20, 120, 220) koaxial ist, wobei der Magnet radial magnetisiert ist und eine Vielzahl radialer Pole definiert, wobei der Magnet die einzige Magnetkomponente innerhalb des Rotors ist.

2. Rotor (10, 110, 210) nach Anspruch 1, wobei die Vielzahl von Rotorschaufeln (30, 130) einen Sammelbereich an einem Außenumfang des Rotors definiert, der von der Nabe (20, 120, 220) entfernt ist, und wobei die Strömungskanäle (12) einen Sammelbereich an dem Außenumfang definieren, und wobei der Sammelbereich, der durch die Vielzahl von Rotorschaufeln (30, 130) an dem Außenumfang definiert ist, größer ist als der Sammelbereich, der durch die Strömungskanäle (12) an dem Außenumfang definiert ist.

3. Rotor (10, 110, 210) nach Anspruch 1, wobei der Magnet (40, 140) zylindrisch ist.

4. Rotor (10, 110, 210) nach einem der Ansprüche 1 bis 3, wobei die Nabe (20, 120, 220) verjüngte Endabschnitte (24, 26) und einen zwischen den Endabschnitten angeordneten Zwischenabschnitt (22) einschließt, wobei der Zwischenabschnitt den Magneten (40, 140) beherbergt und die Rotorschaufeln (30, 130) sich von dem Zwischenabschnitt erstrecken.

5. Rotor (10, 110, 210) nach einem der Ansprüche 1 bis 4, wobei der Magnet (40, 140) Neodym einschließt.

6. Rotor (10, 110, 210) nach einem der Ansprüche 1 bis 5, wobei die Vielzahl von Rotorschaufeln (30, 130) und die Nabe (20, 120, 220) nicht ferromagnetisch sind.

7. Rotor (10, 110, 210) nach einem der Ansprüche 1 bis 6, wobei die Vielzahl von Rotorschaufeln (30, 130) und Nabe (20, 120, 220) aus einem Polymermaterial hergestellt sind.

8. Rotor (10, 110, 210) nach einem der Ansprüche 1 bis 7, wobei die Vielzahl von Rotorschaufeln (30, 130) und Nabe (20, 120, 220) aus einem biokompatiblen Material hergestellt sind und der Magnet (40, 140) ein nicht biokompatibles Material einschließt.

9. Rotor (10, 110, 210) nach einem der Ansprüche 1 bis 8, wobei der Magnet (40, 140) innerhalb des Rotors umschlossen ist.

10. Rotor (10, 110, 210) nach Anspruch 9, wobei der Magnet (40, 140) innerhalb des Rotors abgedichtet ist.

11. Rotor (10, 110, 210) nach einem der Ansprüche 1 bis 10, wobei die Nabe (20, 120, 220) bemessen ist, um innerhalb eines menschlichen Patienten aufgenommen zu werden.

12. Rotor (10, 110, 210) nach einem der Ansprüche 1 bis 11, wobei der Rotor bemessen ist, um innerhalb einer implantierbaren Blutpumpe (50) aufgenommen zu werden.

## Revendications

1. Rotor de pompe (10, 110, 210), comprenant :
un moyeu (20, 120, 220) définissant un axe longitudinal principal ;
un aimant (40, 140) disposé à l'intérieur du moyeu le long de l'axe longitudinal principal ; et
une pluralité de pales de rotor (30, 130) faisant saillie vers l'extérieur à partir du moyeu en s'éloignant de l'axe longitudinal et étant espacées les unes des autres dans une direction circonférentielle autour de l'axe longitudinal, chacune de la pluralité de pales de rotor (30, 130) définissant un palier hydrodynamique (32, 34, 132, 134) à une extrémité externe de celle-ci à distance du moyeu (20, 120, 220), la pluralité de pales de rotor (30, 130) définissant une pluralité de canaux d'écoulement (12), chacune de la pluralité de pales de rotor (30, 130) étant conçue pour entraîner un fluide à travers les canaux d'écoulement lors de la rotation du rotor autour de l'axe, l'aimant (40, 140) est un solide unitaire et est coaxial au moyeu (20, 120, 220), l'aimant étant magnétisé radialement et définissant une pluralité de pôles radiaux, l'aimant étant le seul composant magnétique au sein du rotor.

2. Rotor (10, 110, 210) selon la revendication 1, dans lequel la pluralité de pales de rotor (30, 130) définissent une zone collective au niveau d'une périphérie externe du rotor à distance du moyeu (20, 120, 220), et dans lequel les canaux d'écoulement (12) définissent une zone collective au niveau de la périphérie externe, et dans lequel la zone collective définie par la pluralité de pales de rotor (30, 130) au niveau de la périphérie externe est supérieure à la zone collective définie par les canaux d'écoulement (12) au niveau de la périphérie externe.

3. Rotor (10, 110, 210) selon la revendication 1, dans lequel l'aimant (40, 140) est cylindrique.

4. Rotor (10, 110, 210) selon l'une quelconque des revendications 1 à 3, dans lequel le moyeu (20, 120, 220) comporte des parties d'extrémité effilées (24, 26) et une partie intermédiaire (22) disposée entre les parties d'extrémité, la partie intermédiaire loge l'aimant (40, 140) et les pales de rotor (30, 130) s'étendent à partir de la partie intermédiaire.

5. Rotor (10, 110, 210) selon l'une quelconque des revendications 1 à 4, dans lequel l'aimant (40, 140) comporte du néodyme.

6. Rotor (10, 110, 210) selon l'une quelconque des revendications 1 à 5, dans lequel la pluralité de pales de rotor (30, 130) et le moyeu (20, 120, 220) sont non ferromagnétiques.

7. Rotor (10, 110, 210) selon l'une quelconque des revendications 1 à 6, dans lequel la pluralité de pales de rotor (30, 130) et le moyeu (20, 120, 220) sont réalisés en un matériau polymère.

8. Rotor (10, 110, 210) selon l'une quelconque des revendications 1 à 7, dans lequel la pluralité de pales de rotor (30, 130) et le moyeu (20, 120, 220) sont réalisés en un matériau biocompatible et l'aimant (40, 140) comporte un matériau non biocompatible.

9. Rotor (10, 110, 210) selon l'une quelconque des revendications 1 à 8, dans lequel l'aimant (40, 140) est enfermé au sein du rotor.

10. Rotor (10, 110, 210) selon la revendication 9, dans lequel l'aimant (40, 140) est scellé au sein du rotor.

11. Rotor (10, 110, 210) selon l'une quelconque des revendications 1 à 10, dans lequel le moyeu (20, 120, 220) est dimensionné pour être reçu au sein d'un patient humain.

12. Rotor (10, 110, 210) selon l'une quelconque des revendications 1 à 11, dans lequel le rotor est dimensionné pour être reçu au sein d'une pompe à sang implantable (50).
